# EUROPÄISCHE PATENTSCHRIFT

(11) **EP 0 336 965 B1**
(45) Veröffentlichungstag und Bekanntmachung des Hinweises auf die Patenterteilung: **24.11.1993**
(21) Anmeldenummer: 88906227.9
(22) Anmeldetag: 26.05.1988
(51) Int. Cl.: A61B 17/11

(54) **Gerät zum Anlegen von oesophageal-intestinalen Anastomosen**
Device for performing esophageal-intestinal anastomosis
Dispositif pour la pose d'anastomoses oesophagiens-intestinales

(30) Priorität: 10.06.1987 SU 4303545
(43) Veröffentlichungstag der Anmeldung: 18.10.1989
(73) Patentinhaber: MOSKOVSKY GORODSKOI NAUCHNO-ISSLEDOVATELSKY INSTITUT SKOROI POMOSCHI IMENI N.V. SKLIFOSOVSKOGO, Moscow, 129010 (SU)
(72) Erfinder: KANSHIN, Nikolai Nikolaevich, Moscow, 121433 (SU); LIPATOV, Viktor Alexeevich, Moscow, 117279 (SU); GUSKOV, Igor Alexeevich, Moskau, 109033 (SU)
(74) Vertreter: Nix, Frank Arnold, Dr.
(86) Internationale Anmeldenummer: SU8800125
(87) Internationale Veröffentlichungsnummer: WO8809644

(56) Entgegenhaltungen:
- EP-A- 0 137 685
- SU-A- 1 082 409
- SU-A- 1 158 176
- US-A- 1 918 890
- US-A- 3 771 526
- US-A- 3 789 847

## Beschreibung

Die vorliegende Erfindung bezieht sich auf Einrichtungen zum Verbinden von röhrenförmigen Organen, insbesondere auf Geräte zum Anlegen von oesophageal -intestinalen Anastomosen.

### Zugrundeliegender Stand der Technik

Bereits bekannt sind Nähgeräte zum Anlegen von intestinalen Anastomosen, durch welche eine Klammernaht angelegt wird. Diese Nähgeräte ermöglichen es nicht, die erforderliche Luftdichtheit der angelegten Klammernaht zu erreichen, die bei Operationen am Darm-Kanal von besonderer Notwendigkeit ist, außerdem weist die Klammernaht keine ausreichenden hämostatischen und aseptischen Eigenschaften auf. Bekannt ist weiter eine Vorrichtung zum Verbinden von röhrenförmigen Organen, die, teleskopisch miteinander verbunden, einen zylindrischen Außen- und Innenbecher mit axialen Bohrungen in deren Böden und einen elastischen Ring aufweist, der auf die Außenfläche des Innenbechers aufgesetzt ist. Der Außenbecher ist mit einem koaxialen, röhrenförmigen Handgriff versehen, der ein hohles Gehäuse darstellt und mit der Axialbohrung im Boden des Außenbechers verbunden ist. Die Einrichtung enthält weiter eine Sonde und eine Hülse mit einer Ringnut (SU-A-1158176). Der Innenbecher ragt aus dem Außenbecher zur Bildung eines Mantelflächenabschnittes heraus, auf dem der elastische Ring vor dem Anlegen der Anastomose aufgesetzt wird. Beim Anlegen der Anastomose werden die zu verbindenden Gewebe auf die Ringnut der Hülse gelegt und mit dem elastischen Ring zusammengedrückt. Die Sonde verläuft durch die Hohlräume aller Elemente und ist als hohles Rohr ausgeführt. Auf der Sonde ist mittels einer Tabaksbeutelnaht das eine zu verbindende Organ, und zwar der Darm, festgelegt, während das andere Organ die Becher umfaßt.

Jedoch können bei der Benutzung der bekannten Vorrichtung beim Eintauchen des nicht fixierten Darmes in die Höhle des Innenbechers die Gewebefalten, die zwischen den Boden des Innenbechers und die Hülse gelangen, infolge eines willkürlichen Eintauchens des Darmes in den Innenbecher und wegen der möglichen Versetzung der Tabaksbeutelnaht unterschiedliche Dicke aufweisen, wodurch die Hülse gegenüber dem Innenbecher verschiedene Stellungen einnimmt und beim Hinwegschieben des elastischen Ringes dieser nicht immer zwischen die Bünde der Hülse gelangt, sondern sich auf einen der Bünde legen kann, wodurch die Naht nicht zustandekommt. Das ständige Verbleiben der Sonde innerhalb der Hülse bis zum Augenblick der Abstoßung der abgestorbenen, zusammengedrückten Gewebe des Darmes und der Speiseröhre bewirkt das Ansammeln infizierten Inhaltes innerhalb der Speiseröhrenhöhle mit der Möglichkeit eines postoperativen Durchbruchs des erwähnten Inhaltes in die Atemwege, sodaß ernsthafte Komplikationen der Nachoperationsperiode auftreten können.

### Offenbarung der Erfindung

Der Erfindung liegt die Aufgabe zugrunde, ein Gerät zum Anlegen der oesophageal-intestinalen Anastomosen zu entwickeln, das beim Anlegen der Anastomose durch Änderung der Bauart der gesamten Baugruppe zum Anlegen der Anastomose eine gegenseitige Orientierung des elastischen Ringes und der Hülse gewährleistet. Diese Aufgabe wird dadurch gelöst, daß das Gerät zum Anlegen der oesophageal-intestinalen Anastomosen mit einem hohlen Tragkörper, einem mit diesem hohlen Tragkörper verbundenen Außen- und Innenbecher, die miteinander teleskopisch gekoppelt sind und von denen der Innenbecher aus dem Außenbecher herausragt, um einen Abschnitt zum Aufsetzen eines elastischen, die zu verbindenden biologischen Gewebe zusammendrückenden Rings zu bilden, und jeder Becher mit einer Bohrung in seinem Boden versehen ist, mit deren Hilfe der jeweilige Becher mit dem hohlen Tragkörper in Verbindung steht, einer zur Einführung ins Innere eines der zu verbindenden Hohlorgane vorgesehenen Hülse, welche zur Bildung der Anastomose vermittels des elastischen Ring es auf ihrer Mantelfläche eine erste Ringausfräsung aufweist, und einer mit der Hülse gekoppelten Sonde, die durch die Innenräume der Hülse selbst, der Becher und des hohlen Tragkörpers verläuft, erfindungsgemaß eine zweite Hülse mit einer Ringausfräsung auf ihrer Mantelfläche zur Festlegung des Endes des anderen Verbindungsorgans des oesophageal-intestinalen Traktes auf dieser vermittels einer Tabaksbeutelnaht aufweist, wobei die erste Hülse mit einer zweiten Ringausfräsung auf ihrer Mantelfläche zur Festlegung des Endes des ersten Verbindungsorgans vermittels einer Tabaksbeutelnaht versehen ist und gegen einen an der Sonde ausgeführten Bund stößt, was es erlaubt, bei dem Anlegen der Anastomose durch Verstellung der Sonde die Hülsen zu einer einheitlichen Gruppe zu vereinen, diese ins Innere der Becher hineinzuschieben und hiernach unter Hineinziehung des Innenbechers den elastischen Ring von der Mantelfläche des Innenbechers abzuwerfen, um die zu verbindenden Organe auf der ersten Ringausfräsung der ersten Hülse zusammenzudrücken.

Zweckmäßigerweise hat die Sonde auf ihrem im Inneren der ersten und zweiten Hülse untergebrachten Abschnitt einen mit den Innendurchmessern dieser Hülsen vergleichbaren Durchmesser. Zweckmäßigerweise ist auch ein verstellbarer Anschlagstab an dem hohlen Tragkörper anzuordnen, mit dessen Hilfe der Innenbecher vor dem Anlegen der Anastomose gehalten wird.

### Kurzbeschreibung der Zeichnungen

Nachstehend wird die Erfindung durch die Beschreibung eines Ausführungsbeispiels und die beigelegten Zeichnungen erläutert; in diesen zeigt:
Fig. 1 die Gesamtansicht eines erfindungsgemäßen Geräts zum Anlegen von oesophageal-intestinalen Anastomosen;
Fig. 2 die erfindungsgemäße Baugruppe zum Anlegen der Anastomose im Längsschnitt;
Fig. 3 die gleiche Baugruppe im Augenblick kurz vor dem Abschieben des elastischen Ringes im Längsschnitt;
Fig. 4 die gleiche Baugruppe nach dem Anlegen der Anastomose im Längsschnitt.

### Beste Ausführungsvariante der Erfindung

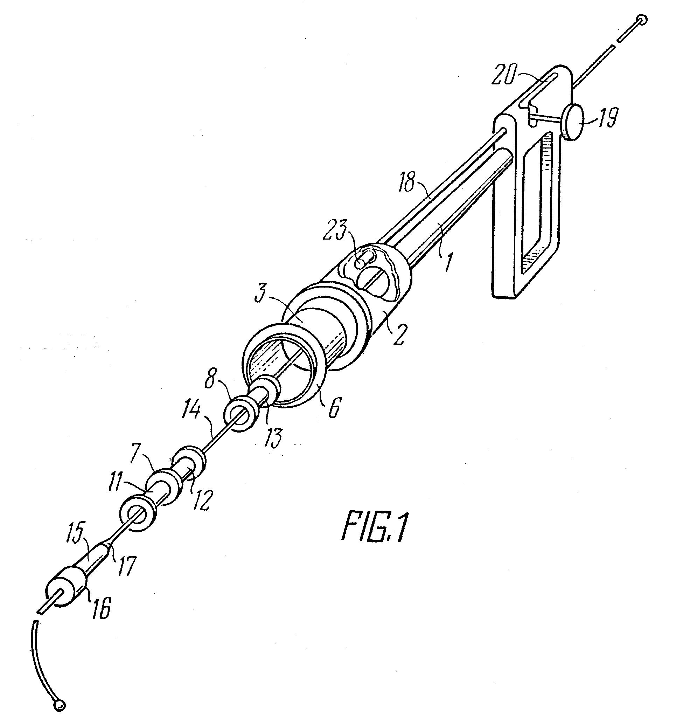

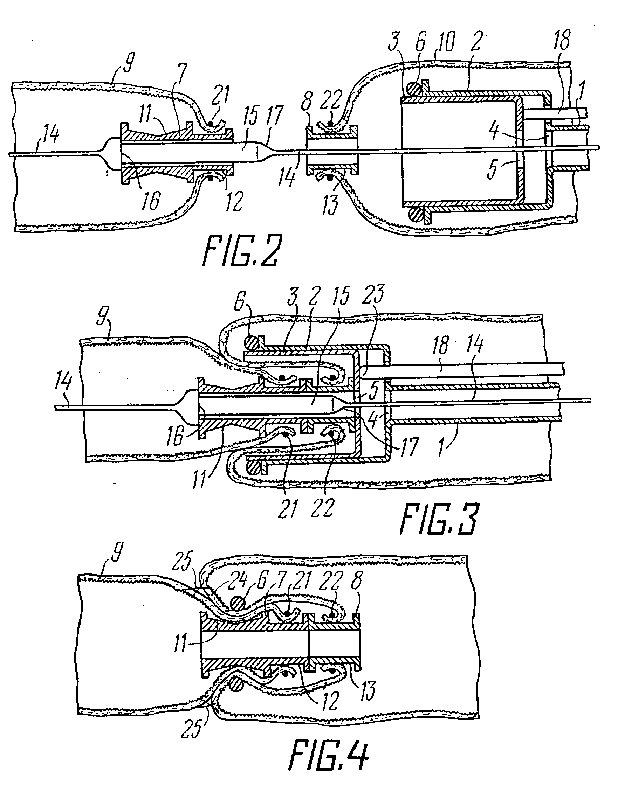

Das erfindungsgemäße Gerät zum Anlegen von oesophageal-intestinalen Anastomosen hat einen hohlen Tragkörper 1 (Fig. 1) und einen Außenbecher 2 und einen Innenbecher 3, die teleskopisch miteinander verbunden sind. Im Boden jedes Bechers 2, 3 ist je eine Bohrung 4 bzw. 5 ausgespart (Fig. 2), durch welche die Innenräume der Becher 2, 3 mit dem Innenraum des mit dem Außenbecher 2 verbundenen Tragkörpers 1 kommunizieren. Auf der Außenmantelfläche des Innenbechers 3 ist ein elastischer Ring 6 (Fig. 1) aufgesetzt, wozu der Innenbecher 3 aus dem Außenbecher 2 hervorragt, sodaß ein Abschnitt der Außenfläche des Innenbechers 3 zur Aufnahme des elastischen Ringes 6 vor dem Anlegen der Anastomose gebildet ist. Das Gerät hat auch Hülsen 7 und 8 (Fig. 1, 2), an denen die Enden der zu verbindenden Organe 9 und 10, beispielsweise der Speiseröhre (9) und des Darms (10) befestigt werden. Die Hülse 7 weist zwei Teilabschnitte mit Ringausfräsungen 11 und 12 in jedem Abschnitt auf. Die Ringausfräsung 11 ist zur Bildung der Anastomose vermittels des elastischen Rings 6 vorgesehen. Die Hülse 8 ist mit nur einer Ringausfräsung 13 zur Befestigung des Verbindungsorgans 10 analog der Befestigung des Verbindungsorgans 9 versehen. Durch die Höhlen der Hülsen 7, 8, Becher 2, 3 und des Tragkörpers 1 verläuft eine Sonde 14, die als ein biegsamer Zugdraht ausgeführt ist. Die Sonde 14 weist einen verdickten Abschnitt 15 auf, der im Bereich der Hülsen 7 und 8 gelegen ist. Der Durchmesser dieses verdickten Abschnitts 15 ist zur Erhaltung der Gleichachsigkeit der Hülsen 7, 8 und demzufolge der koaxialen Anordnung der Verbindungsorgane 9, 10 mit dem Innendurchmesser der Hülsen 7, 8 vergleichbar. Die Sonde 14 hat einen Bund 16, gegen den das Stirnende der Hülse 7 stößt, und ein Kegelende 17 zum bequemeren Durchführen der Sonde 14 durch die Höhlen der Hülsen 7, 8.

An dem hohlen Tragkörper 1 ist noch ein verstellbarer Anschlag 18 in Form eines Stabes mit in einen Schlitz 20 hineinragendem Bajonettenverschluß 19 angebracht. Das erfindungsgemäße Gerät arbeitet wie folgt. Zunächst wird in das resezierte Ende eines Verbindungsorgans 9, beispielsweise der Speiseröhre, die Hülse 7 mit der im voraus in diese hineingesteckten Sonde 14 derart eingeführt, daß dabei der zylindrische verdickte Abschnitt 15 in die Hülse 7 hineingeht, wonach das Ende der Sonde 14 über dem Bund 16 innerhalb der Speiseröhre bis in die Mundhöhle hindurchgezogen wird, während das Randende der Speiseröhre in der Ringausfräsung 12 der Hülse 7 durch eine Ligatur 21 festgesetzt wird. Danach wird die Hülse 8 mit ihrer Ringausfräsung 13 ins Ende des anderen zur Anastomosenanlegung vorgesehenen Organs, hier des Darmes 10, hineingeführt und das Organ 10 mit einer Ligatur 22 auf der Hülse 8 festgelegt. Durch einen Zusatzeinschnitt im Darm wird jetzt das zusammengebaute Gerät bis zur Berührung mit der im Darm gehaltenen Hülse 8 in den Darm 10 eingeführt. Das andere Ende der Sonde 14 (Fig. 2) wird durch die Hülse 8 und durch das ganze Gerät bis seinem Austritt nach außen hindurchgezogen. Im weiteren wird die Sonde 14 verstellt, wobei die Hülsen 7, 8 in einen dichten Kontakt treten und sich zu einer einheitlichen Gruppe zusammenfügen und das festgelegte Organ 10 in den Innenbecher 3 des Gerätes und das Organ 9 in das Organ 10 hineintauchen. Die Sonde 14 wird verstellt, bis die Hülse 8 gegen den Boden des Innenbechers 3 (Fig. 3) stößt.

Hierbei ist das Eindringen der Gewebe der Organe 9 und 10 zwischen den Hülsen 7 und 8, zwischen der Hülse 8 und dem Boden des Innenbechers 3 vermieden, d.h. die Stellungslage der Ringausfräsung 11 der Hülse 7 gegenüber dem elastischen Ring 6 ist gesichert. Mit Hilfe des Bajonettenverschlusses 19 wird das Ende 23 des Anschlagstabes 18 aus dem Innenraum des Außenbechers 2 herausgezogen und durch weiteres Ziehen der Sonde 14 der Innenbecher 3 mit den Hülsen 7 und 8 bis zum Anschlag gegen den Boden des Außenbechers 2 verstellt, wobei der elastische Ring 6 von der Mantelfläche des Innenbechers 3 abgeschoben wird und genau in die Ringausfräsung 11 (Fig. 4) gelangt und die Darmwandung und die Wandung der in den Darm invaginierten Speiseröhre von der Lumenseite zusammendrückt.

In Anbetracht der kürzeren Länge des Innenbechers 3 wird die Speiseröhre zusätzlich in die trichterförmige Falte 24 der Darmwandung hineingezogen, wodurch es möglich wird, die Fläche des Zusammenwachsens der Wandung der Speiseröhre mit der Darmwandung zu vergrößern, sowie beide Wandungen vermittels mehrerer Knotennähte 25 aneinander zu befestigen, die durch die oberflächlichen schichten der Wandungen der Speiseröhre und des Darms angelegt werden. Hiernach wird das erfindungsgemäße Gerät aus dem Darm durch die zusätzliche Hilfsöffnung in diesem herausgenommen, die dann zusammengenäht wird, während die Sonde 14 aus der Mundhöhle herausgezogen wird. Dabei werden die Innenbohrungen der Hülsen 7 und 8 geöffnet, was die Ernährung des Kranken und einen freien Abfluß des Speiseröhreninhalts auf natürlichem Wege ermöglicht. Nach 7 bis 10 Tagen werden die abgestorbenen, durch den elastischen Ring 6 zusammengedrückten Gewebe des Darms und der Speiseröhre samt der Hülsen 7 und 8 und des elastischen Ringes 6 abgestoßen und auf natürlichem Wege entfernt. Mit Hilfe des vorliegenden Geräts wurden 5 Operationen der Vereinigung der Därme von Hunden vorgenommen. Die Operationen endeten erfolgreich, es gab keine Komplikationen. Es sind weiter die Operationen der Magenresektion bei 4 Kranken durchgeführt worden, wobei vermittels des betrachteten Geräts die Speiseröhre mit dem Darm verbunden wurde. Die Anastomose hat sich im Laufe von 8 Tagen gebildet, wonach die Hülsen 7, 8 und der elastische Ring 6 abgestoßen wurden. Die gebildete Naht dehnte sich von selbst auf die Abmessungen der Speiseröhre. Es wurden keine Komplikationen beobachtet. Da das Zusammenwachsen der Wandungen des gut ausgedehnten Darmes und der Speiseröhre unter aseptischen Verhältnissen vor sich geht, wird die Anastomose sicher, läßt sich leicht auseinanderziehen, weist praktisch keine Narbengewebe auf.

### Anwendbarkeit

Die Erfindung kann in der Chirurgie bei der Magenresektion und anderen Operationen mit Anlegung von Anastomosen an Verdauungsorganen ihre Anwendung finden.

## Patentansprüche

1. Gerät zum Anlegen von oesophageal-intestinalen Anastomosen mit einem hohlen Tragkörper (1), einem mit dem hohlen Tragkörper verbundenen Außenbecher (2) und einem teleskopisch mit dem Außenbecher verbundenen Innenbecher (3),
wobei der Innenbecher (3) aus dem Außenbecher (2) unter Bildung eines Mantelflächenabschnitts zur Anordnung eines elastischen, die zu verbindenden biologischen Gewebe beim Anlegen der Anastomose zusammendrückenden Ringes (6) hervorragt und jeder Becher (2, 3) in seinem Boden je eine Bohrung (4, 5) aufweist, über welche der jeweilige Becher (2, 3) mit dem hohlen Tragkörper (1) in Verbindung steht,
mit einer innerhalb eines der zu verbindenden Organe (9) unterzubringenden Hülse (7) mit einer ersten Ringausfräsung (11) an ihrer Mantelfläche zur Bildung der Anastomose mittels des elastischen Ringes (6),
und mit einer Sonde (14), die mit der Hülse (7) verbunden ist und durch diese selbst, die Becher (2, 3) sowie den hohlen Tragkörper (1) verläuft,
dadurch gekennzeichnet, daß es eine zweite Hülse (8) mit einer Ringausfräsung (13) auf der Mantelfläche zur Befestigung des Endes des anderen Verbindungsorganes (10) auf dieser aufweist, und daß die erste Hülse (7) an ihrer Mantelfläche mit einer zweiten Ringausfräsung (12) zur Befestigung des Endes des ersteren Verbindungsorgans (9) in dieser versehen ist und gegen einen an der Sonde (14) vorgesehenen Bund (16) stößt, wodurch es möglich wird, bei der Anlegung der Anastomose durch Verstellung der Sonde (14) die Hülsen (7, 8) zu einer einheitlichen Gruppe zu vereinen, diese ins Innere der Becher (2, 3) hineinzuschieben und hiernach unter Hineinziehen des Innenbechers (3) in den Außenbecher (2) den elastischen Ring (6) von der Außenfläche des Innenbechers (3) abzuschieben, so daß dieser die zu verbindenden Organe (9, 10) auf der ersten Ringausfräsung (11) der ersten Hülse (7) zusammendrückt.

2. Gerät nach Anspruch 1, dadurch gekennzeichnet, daß die Sonde (14) auf dem Abschnitt ihrer Anordnung innerhalb der ersten Hülse (7) und der zweiten Hülse (8) einen mit den Innendurchmessern der Hülsen (7, 8) vergleichbaren Durchmesser aufweist.

3. Gerät nach Anspruch 1 oder 2, dadurch gekennzeichnet, daß an dem hohlen Tragkörper (1) ein verstellbarer Anschlagstab (18) angebracht ist, mit dessen Hilfe der Innenbecher (3) vor dem Anlegen der Anastomose gehalten wird.

## Claims

1. Apparatus for applying oesophagointestinal anastomoses, having a hollow support body (1), an outer cup (2) which is connected to the hollow support body, and an inner cup (3) which is connected telescopically to the outer cup, the inner cup (3) protruding from the outer cup (2) forming a section of the outer surface for the arrangement of an elastic ring (6) which compresses the biological tissues to be connected when the anastomosis is applied, and each cup (2, 3) having a bore (4, 5) in its bottom in each case, via which bore the respective cup (2, 3) communicates with the hollow support body (1), having a bush (7) which is to be accommodated inside one of the organs (9) to be connected and has a first annular recess (11) on its outer surface to form the anastomosis by means of the elastic ring (6), and having a probe (14) which is connected to the bush (7) and runs through the latter itself, the cups (2, 3) and the hollow support body (1), characterised in that said apparatus has a second bush (8) with an annular recess (13) on the outer surface for attaching the end of the other connection organ (10) thereon, and in that the first bush (7) is provided on its outer surface with a second annular recess (12) for attaching the end of the former connection organ (9) therein and strikes against a collar (16) provided on the probe (14), as a result of which it is possible, when the anastomosis is applied by adjusting the probe (14), to combine the bushes (7, 8) to form a uniform group, to insert them inside the cups (2, 3) and then to push the elastic ring (6) off the outer surface of the inner cup (3) whilst drawing the inner cup (3) into the outer cup (2) so that said ring compresses the organs (9, 10) to be connected on the first annular recess (11) of the first bush (7).

2. Apparatus according to Claim 1, characterised in that, on the section of its arrangement inside the first bush (7) and the second bush (8), the probe (14) has a diameter which is comparable to the inside diameters of the bushes (7, 8).

3. Apparatus according to Claim 1 or 2, characterised in that an adjustable stop bar (18) is mounted on the hollow support body (1), with the aid of which stop bar the inner cup (3) is held before the anastomosis is applied.

## Revendications

1. Appareil pour effectuer des anastomoses oesophagiennes-intestinales comprenant un corps de support creux (1), un gobelet externe (2) relié au corps de support creux et un gobelet interne (3) relié télescopiquement avec le gobelet externe, le gobelet interne (3) faisant saillie du gobelet externe (2) en formant un tronçon de surface latérale pour disposer une bague élastique (6) comprimant les tissus biologiques à relier lors de la réalisation de l'anastomose, et chaque gobelet (2, 3) présentant dans son fond, respectivement, un perçage (4, 5) par lequel le gobelet respectif (2, 3) est en liaison avec le corps de support creux (1), avec un manchon (7) à insérer à l'intérieur d'un des organes à relier (9) avec un premier fraisage annulaire (11) sur sa surface latérale pour former l'anastomose au moyen de la bague élastique (6), et avec une sonde (14) qui est reliée au manchon (7) et qui s'étend à travers celui-ci, les gobelets (2, 3) ainsi que le corps de support creux (1),
caractérisé en ce qu'il présente un deuxième manchon (8) comprenant un fraisage annulaire (13) sur la surface latérale pour fixer l'extrémité de l'autre organe de liaison (10) sur celle-ci, et en ce que le premier manchon (7) est pourvu sur sa surface latérale d'un deuxième fraisage annulaire (12) pour fixer l'extrémité du premier organe de liaison (9) dans celle-ci et bute contre un collet (16) prévu à la sonde (14), par quoi il devient possible lors de la réalisation de l'anastomose, de réunir par un déplacement de la sonde (14) les manchons (7, 8) en un groupe uniforme, de pousser celui-ci dans l'intérieur des gobelets (2, 3) et de pousser ensuite, en tirant le gobelet interne (3) dans le gobelet externe (2), la bague élastique (6) de la surface externe du gobelet interne (3) de façon que celle-ci comprime les organes à relier (9,10) sur le premier fraisage annulaire (11) du premier manchon (7).

2. Appareil selon la revendication 1, caractérisé en ce que la sonde (14) présente sur le tronçon de sa disposition à l'intérieur du premier manchon (7) et du deuxième manchon (8), un diamètre comparable aux diamètres internes des manchons (7, 8).

3. Appareil selon la revendication 1 ou 2, caractérisé en ce qu'il est prévu au corps de support creux (1) une tige de butée réglable (18) à l'aide de laquelle le gobelet interne (3) est maintenu avant la réalisation de l'anastomose.
